# EUROPÄISCHE PATENTSCHRIFT

(11) **EP 2 653 122 B1**
(45) Veröffentlichungstag und Bekanntmachung des Hinweises auf die Patenterteilung: **08.02.2017**
(21) Anmeldenummer: 13001866.6
(22) Anmeldetag: 11.04.2013
(51) Int. Cl.: A61B 17/295, A61B 17/29

(54) **Werkzeug für ein medizinisches Instrument**
Tool for a medical instrument
Outil pour un instrument médical

(30) Priorität: 18.04.2012 DE 102012007647
(43) Veröffentlichungstag der Anmeldung: 23.10.2013
(73) Patentinhaber: Karl Storz GmbH & Co. KG, 78532 Tuttlingen (DE)
(72) Erfinder: Blocher, Martin, D-78532 Tuttlingen (DE); Merz, Robin, D-78532 Tuttlingen (DE)

(56) Entgegenhaltungen:
- EP-A2- 1 468 653
- EP-A2- 1 927 320
- EP-A2- 2 077 773
- DE-U1-202007 009 315
- US-A- 5 673 841
- US-A- 5 683 359
- US-B2- 7 976 552

## Beschreibung

Die vorliegende Erfindung ist auf ein Werkzeug für ein distales Ende oder an einem distalen Ende eines Schafts eines medizinischen Instruments und auf ein medizinisches Instrument mit einem solchen Werkzeug bezogen.

Medizinische Einrichtungen und medizinisches Personal bevorzugen möglichst vielfältig verwendbare medizinische Instrumente. Je vielfältiger das einzelne medizinische Instrument einsetzbar ist, desto geringer ist die Vielfalt der bereitzuhaltenden Instrumente. Vielfältig einsetzbare medizinische Instrumente können deshalb auch bei höheren Stückkosten die in einer medizinischen Einrichtung erforderlichen Investitionen senken. Durch eine geringe Vielfalt und eine geringere Stückzahl der bereitzuhaltenden medizinischen Instrumente können ferner die Kosten für Lagerhaltung und Logistik verringert werden. Bei mikroinvasiven medizinischen Instrumenten bzw. medizinischen Instrumenten für mikroinvasive Eingriffe kommt hinzu, dass während eines Eingriffs umso seltener Instrumente gewechselt werden müssen, je vielfältiger das einzelne Instrument einsetzbar ist. Eine Aufgabe der vorliegenden Erfindung besteht darin, ein verbessertes Werkzeug für ein distales Ende oder an einem distalen Ende eines medizinischen Instruments und ein verbessertes medizinisches Instrument zu schaffen. Insbesondere besteht eine Aufgabe darin, bei einem Werkzeug mit einem schwenkbaren Maulteil einen Arbeitskanal zu ermöglichen, in dem ein Skalpell oder ein anderes Wirkelement angeordnet und bewegt werden kann.

In EP 1 468 653 A2, auf welcher der Oberbegriff von Anspruch 1 beruht, ist eine Vorrichtung zum Applizieren flexibler chirurgischer Clips beschrieben. Die Vorrichtung umfasst zwei bewegliche Maulteile, die über Gelenke mit einem Gabelkopf verbunden sind. Im Gabelkopf ist ein Kanal vorgesehen, durch den elastische chirurgische Clips nach distal in den Raum zwischen den Maulteilen geschoben werden können.

In EP 1 927 320 A2 ist ein medizinisches Instrument mit einem starren Maulteil und einem schwenkbaren Maulteil beschrieben. Am schwenkbaren Maulteil sind zwei Lagerzapfen vorgesehen, die in korrespondierende Lageraufnahmen eingreifen.

In EP 2 077 773 B1 ist ein medizinisches Instrument mit einem feststehenden Maulteil und einem schwenkbaren Maulteil beschrieben. Am schwenkbaren Maulteil sind zwei gebogene Gelenkführungsschienen vorgesehen, die durch zwei Gelenkführungszapfen am starren Maulteil so geführt sind, dass eine virtuelle Drehgelenksachse sich außerhalb der Maulteile befindet.

In US 5,673,841 ist ein chirurgisches Instrument für endoskopisch chirurgische Verfahren beschrieben. Eine schwenkbare Backe kann durch Wechselwirkung eines Schlitzes und einer Oberfläche an einer Einrichtung mit einem Stift bzw. einer Fläche an der Backe bewegt werden.

In US 7,976,552 B2 ist eine Vorrichtung zur Ligatur beschrieben. Zwei Klemmteile sind durch Gelenke mit einem rohrförmigen Bauteil verbunden, in dem eine Clipeinheit bewegt werden kann.

In DE 20 2007 009 315 U1 ist ein chirurgisches Mikrorohrschaftinstrument mit schwenkbaren Schafthalbschalen beschrieben. Durch Longitudinalbewegung eines rohrförmigen Außenschafts, an dessen distalem Ende ein Ring angeordnet ist, relativ zu den Schafthalbschalen können diese geschwenkt werden.

Diese Aufgabe wird durch die Gegenstände der unabhängigen Ansprüche gelöst.

Weiterbildungen sind in den abhängigen Ansprüchen angegeben.

Ein Werkzeug für ein distales Ende oder an einem distalen Ende eines Schafts eines medizinisches Instruments umfasst einen Grundkörper, ein Maulteil, das relativ zum Grundkörper um eine Schwenkachse schwenkbar ist, und ein Lager, das die Schwenkachse definiert, wobei das Lager zwei voneinander beabstandete Achszapfen mit je einer zur Schwenkachse rotationssymmetrischen Mantelfläche umfasst.

Das Werkzeug ist zur (insbesondere lösbaren) mechanischen Kopplung mit dem distalen Ende eines Schafts ausgebildet oder mit dem distalen Ende eines Schafts (insbesondere nicht zerstörungsfrei lösbar) mechanisch verbunden. Dabei ist der Schaft Bestandteil eines medizinischen Instruments oder zur Bildung eines medizinischen Instruments vorgesehen.

Der Grundkörper weist insbesondere am proximalen Ende oder in einem proximalen Bereich eine Kupplung zur lösbaren mechanischen Kopplung mit einem distalen Ende eines Schafts auf. Alternativ ist der Grundkörper mit dem distalen Ende eines Schafts dauerhaft und nicht zerstörungsfrei lösbar mechanisch verbunden. Das Werkzeug kann ein weiteres Maulteil aufweisen, das gegenüber dem Grundkörper nicht bewegbar ist. Dieses weitere Maulteil ist insbesondere mit dem Grundkörper starr verbunden oder mit ihm einstückig ausgebildet. Alternativ oder zusätzlich kann das Werkzeug ein oder mehrere weitere bewegbare, insbesondere schwenkbare, Maulteile aufweisen.

Das Lager kann mehr als zwei voneinander beabstandete Achszapfen mit je einer zur Schwenkachse des Maulteils rotationssymmetrischen Mantelfläche umfassen. Jeder einzelne Achszapfen ist entweder mit dem schwenkbaren Maulteil oder mit dem Grundkörper starr verbunden.

Wie im Folgenden beschrieben und anhand der Ausführungsbeispiele im Detail dargestellt ist, schaffen die zwei (oder mehr) voneinander beabstandeten Achszapfen neue konstruktive Freiheitsgrade.

Ein Werkzeug, wie es hier beschrieben ist, umfasst insbesondere ferner einen Arbeitskanal, in dem ein Skalpell oder ein anderes Wirkelement zwischen einer proximalen und einer distalen Position verschiebbar ist.

Das Skalpell ist insbesondere zum Durchtrennen von mittels des Werkzeugs gefasstem Gewebe vorgesehen und ausgebildet. Beispielsweise ist das Werkzeug zur monopolaren oder bipolaren Elektrokauterisation von mittels des Werkzeugs gefasstem Gewebe ausgebildet. Ein Gefäß oder anderes Gewebe kann mittels des Werkzeugs gegriffen, gequetscht, elektrochirurgisch bzw. HF-chirurgisch verödet und dann mittels des Skalpells durchtrennt werden. Die Durchführbarkeit aller dieser Verfahrensschritte mit einem einzigen Werkzeug kann den Zeitaufwand eines mikrochirurgischen Eingriffs deutlich mindern. Der Arbeitskanal kann statt für ein Skalpell für andere Wirkelemente verwendbar sein, die bei einer offenen und/oder bei einer geschlossenen Position des Werkzeugs eingesetzt werden können.

Bei einem Werkzeug mit einem Arbeitskanal, wie es hier beschrieben ist, ist der Arbeitskanal insbesondere zwischen den Achszapfen angeordnet.

Der Arbeitskanal erstreckt sich insbesondere von einer Handhabungseinrichtung entlang eines Schafts bis zum Werkzeug und zum Wirkbereich des schwenkbaren Maulteils. Eine Anordnung des Arbeitskanals zwischen den Achszapfen bedeutet, dass der Arbeitskanal zwischen den Achszapfen hindurchführt bzw. zumindest ein Teil des Lumens des Arbeitskanals zwischen den Achszapfen liegt.

Das Lager mit zwei (oder mehr) voneinander beabstandeten Achszapfen ermöglicht bei einer nahezu beliebigen Anordnung der Schwenkachse des schwenkbaren Maulteils einen zumindest in Richtung senkrecht zur Längsachse des Werkzeugs und senkrecht zur Schenkachse großen Querschnitt bzw. ein großes Lumen des Arbeitskanals. Vergleichbare Querschnitte des Arbeitskanals sind im Falle eines Lagers mit einer durchgehenden Welle allenfalls bei einer Anordnung der Welle mit großem Abstand zur Mitte des Querschnitts und mit kleinem Abstand der Welle zum äußeren Rand des Querschnitts des Werkzeugs möglich. Diese Anordnung der Welle ist bei einem kreisförmigen Querschnitt des Werkzeugs jedoch nur möglich, wenn die Welle kurz ist, was andere konstruktive Nachteile mit sich bringt.

Bei einem Werkzeug, wie es hier beschrieben ist, umfasst der Grundkörper des Werkzeugs insbesondere zwei parallele Wände, zwischen denen ein Abschnitt des Arbeitskanals angeordnet ist, wobei jede der zwei parallelen Wände eine Ausnehmung aufweist, in die einer der zwei Achszapfen eingreift.

Die zwei parallelen Wände sind insbesondere als Stege an einer Grundplatte ausgebildet.

Die zwei (oder mehr) parallelen Wände sind insbesondere parallel zur Längsachse des Werkzeugs bzw. eines mit dem Werkzeug verbundenen oder zu verbindenden Schaft und senkrecht zur Schwenkachse des schwenkbaren Maulteils angeordnet. Die zwei parallelen Wände begrenzen insbesondere den Arbeitskanal an zwei gegenüberliegenden Seiten, so dass der Arbeitskanal zumindest abschnittsweise durch die zwei gegenüberliegenden Wände begrenzt ist. Jede Ausnehmung wird insbesondere durch ein Sackloch in einer der zwei parallelen Wände gebildet. Alternativ werden beide Ausnehmungen durch eine einzige, beide parallele Wände durchdringende Durchgangsbohrung, gebildet.

Bei einem Werkzeug, wie es hier beschrieben ist, umfasst das Lager insbesondere ferner zu den Achszapfen korrespondierende Ausnehmungen, wobei die Achszapfen von außen in die Ausnehmungen eingreifen.

Wie bereits für die Ausführungsform mit zwei parallelen Wänden erwähnt, können auch in anderen Fällen die zu den Achszapfen korrespondierenden Ausnehmungen durch eine einzige Durchgangsbohrung gebildet werden, wobei die Achszapfen in die beiden gegenüberliegenden Enden der Durchgangsbohrung eingreifen. Mit einem Eingreifen von außen ist ein Eingreifen aus zwei entgegengesetzten Richtungen gemeint, bei dem die Achszapfen auf einander zu ragen.

Bei einem Werkzeug, wie es hier beschrieben ist, umfasst das Lager insbesondere ferner zu den Achszapfen korrespondierende Ausnehmungen, wobei die Achszapfen von innen in die Ausnehmungen eingreifen.

Mit einem Eingreifen der Achszapfen in die Ausnehmungen von innen ist gemeint, dass die Achszapfen in zwei voneinander abgewandte entgegengesetzte Richtungen vorragen.

Bei einem Werkzeug, wie es hier beschrieben ist, umfasst das Maulteil insbesondere zwei Achszapfenbauteile mit je einem der Achszapfen.

Jedes Achszapfenbauteil ist insbesondere einstückig gefertigt. Jedes Achszapfenbauteil umfasst insbesondere an seinem proximalen Ende den zugeordneten Achszapfen und einen Arm bzw. Holm, der sich im Wesentlichen senkrecht zur Schwenkachse des schwenkbaren Maulteils erstreckt. Wenn die Achszapfenbauteile Bestandteil des schwenkbaren Maulteils sind, sind die Arme bzw. Holme insbesondere mit dem schwenkbaren Maulteil verbunden, in diesem angeordnet oder bilden das schwenkbare Maulteil.

Alternativ sind die Arme bzw. Holme mit dem Grundkörper des Werkzeugs starr verbunden, und die Achszapfen greifen in Ausnehmungen ein, die an dem schwenkbaren Maulteil vorgesehen sind.

Bei einem Werkzeug mit zwei Achszapfenbauteilen, wie es hier beschrieben ist, wurden insbesondere die Achszapfenbauteile nach einem Einsetzen der Achszapfen in korrespondierende Ausnehmungen miteinander starr verbunden.

Insbesondere wurden nach dem Einsetzen der Achszapfen in korrespondierende Ausnehmungen die bereits erwähnten Arme bzw. Holme mittelbar oder unmittelbar miteinander gefügt, um beispielsweise das schwenkbare Maulteil zu bilden.

Bei einem Werkzeug mit Achszapfenbauteilen, die nach einem Einsetzen der Achszapfen in korrespondierende Ausnehmungen miteinander starr verbunden wurden, sind die Achszapfenbauteile insbesondere mittels eines Grundkörpers des schwenkbaren Maulteils miteinander starr verbunden.

Insbesondere sind die erwähnten Arme bzw. Holme der Achszapfenbauteile in Bohrungen oder Nuten im Grundkörper des schwenkbaren Maulteils eingesetzt und gefügt.

Ein Werkzeug, wie es hier beschrieben ist, umfasst insbesondere ferner eine Übertragungseinrichtung zum Übertragen einer Kraft zum Werkzeug, wobei die Übertragungseinrichtung und das schwenkbare Maulteil aneinander anliegende gekrümmte Gleitflächen aufweisen, die dazu ausgebildet sind, eine Translationsbewegung der Übertragungseinrichtung mit einer Schwenkbewegung des schwenkbaren Maulteils zu koppeln, wobei ein Krümmungsmittelpunkt der gekrümmten Gleitflächen außerhalb des Werkzeugs liegt.

Die Übertragungseinrichtung ist insbesondere längs eines mit dem Werkzeug koppelbaren oder dauerhaft verbundenen Schafts verschiebbar. Die Übertragungseinrichtung kann mittels der aneinander anliegenden gekrümmten Gleitflächen derart mit dem Maulteil gekoppelt sein, dass eine Bewegung der Übertragungseinrichtung nach proximal eine schließende Schwenkbewegung des Maulteils oder alternativ umgekehrt eine öffnende Bewegung des Maulteils bewirkt. Die Übertragungseinrichtung ist dabei insbesondere innerhalb des Werkzeugs oder innerhalb eines mit dem Werkzeug koppelbaren oder dauerhaft verbundenen Schafts so geführt, dass sie entlang eines im Wesentlichen geraden oder leicht gekrümmten Pfads verschiebbar ist.

Die jeweils paarweise aneinander anliegenden Gleitflächen der Übertragungseinrichtung und des schwenkbaren Maulteils weisen insbesondere einen gemeinsamen Krümmungsmittelpunkt auf oder sind rotationssymmetrisch zu einer Achse. Diese im Wesentlichen kreisbogenförmige Gestalt der Gleitflächen ermöglicht einen flächigen Kontakt, der zur Übertragung großer Kräfte geeignet sein kann.

Alternativ können die Gleitflächen mehrere Krümmungsmittelpunkte bzw. eine variierende Krümmung bzw. eine nicht zu einer Achse rotationssymmetrische Gestalt aufweisen. Mit dieser im Ergebnis nicht kreisbogenförmigen Gestalt der Gleitflächen kann ein variierendes Übersetzungsverhältnis zwischen der Bewegung der Übertragungseinrichtung und der Schwenkbewegung des schwenkbaren Maulteils realisiert werden.

Die Anordnung des Krümmungsmittelpunkts der gekrümmten Gleitflächen ermöglicht ein großes Übersetzungsverhältnis, bei dem für eine vorbestimmte Schwenkbewegung des schwenkbaren Maulteils eine Translationsbewegung der Übertragungseinrichtung entlang eines verhältnismäßig langen Wegs erforderlich ist.

Bei einem Werkzeug mit aneinander anliegenden gekrümmten Gleitflächen an Übertragungseinrichtung und schwenkbarem Maulteil, wie es hier beschrieben ist, sind insbesondere die gekrümmten Gleitflächen am Maulteil parallele Flächen an einer gekrümmten Nut und die gekrümmten Gleitflächen an der Übertragungseinrichtung parallele Flächen an einem gekrümmten Steg. Alternativ können die gekrümmten Gleitflächen am Maulteil parallele Flächen an einem gekrümmten Steg und die gekrümmten Gleitflächen an der Übertragungseinrichtung parallele Flächen an einer gekrümmten Nut sein. Alternativ können die gekrümmten Gleitflächen nicht-parallel sein.

Bei einem Werkzeug, wie es hier beschrieben ist, liegt die Schwenkachse, um die das schwenkbar gelagerte Maulteil schwenkbar ist, insbesondere innerhalb des Werkzeugs.

Mit einer Anordnung der Schwenkachse innerhalb des Werkzeugs ist gemeint, dass die Schwenkachse das von dem Werkzeug eingenommene Volumen schneidet bzw. durchstößt. Anders ausgedrückt liegt ein Abschnitt der Schwenkachse innerhalb der Kontur des Werkzeugs.

Bei einem Werkzeug, wie es hier beschrieben ist, ist insbesondere an zumindest einem der zwei Achszapfen eine Nut innerhalb eines kreiszylindrischen Raumbereichs vorgesehen, wobei die Mantelfläche der Achszapfen auf einer Mantelfläche des kreiszylindrischen Raumbereichs liegt.

Einander gegenüberliegende Seitenwände der Nut bilden insbesondere die erwähnten gekrümmten Gleitflächen am schwenkbaren Maulteil. In die Nut greift insbesondere ein Steg an der erwähnten Übertragungseinrichtung ein.

Ein medizinisches Instrument umfasst ein Werkzeug, wie es hier beschrieben ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Darstellung eines Werkzeugs des medizinischen Instruments aus Figur 1;
- Figur 3: eine schematische axonometrische Darstellung von Teilen des Werkzeugs aus Figur 2;
- Figur 4: eine weitere schematische axonometrische Darstellung von Teilen des Werkzeugs aus den Figuren 2 und 3;
- Figur 5: eine weitere schematische axonometrische Darstellung von Teilen des Werkzeugs aus den Figuren 2 bis 4;
- Figur 6: eine weitere schematische axonometrische Darstellung von Teilen des Werkzeugs aus den Figuren 2 bis 5;
- Figur 7: eine weitere schematische axonometrische Darstellung von Teilen des Werkzeugs aus den Figuren 2 bis 6;
- Figur 8: eine weitere schematische axonometrische Darstellung von Teilen des Werkzeugs aus den Figuren 2 bis 7;
- Figur 9: eine weitere schematische axonometrische Darstellung von Teilen des Werkzeugs aus den Figuren 2 bis 8;
- Figur 10: eine schematische Darstellung eines Schnitts durch das Werkzeug aus den Figuren 2 bis 9;
- Figur 11: eine schematische Darstellung eines weiteren Schnitts durch das Werkzeug aus den Figuren 2 bis 10;
- Figur 12: eine weitere schematische Darstellung von Teilen des Werkzeugs aus den Figuren 2 bis 11;
- Figur 13: eine weitere schematische Darstellung von Teilen des Werkzeugs aus den Figuren 2 bis 12.

### Beschreibung der Ausführungsformen

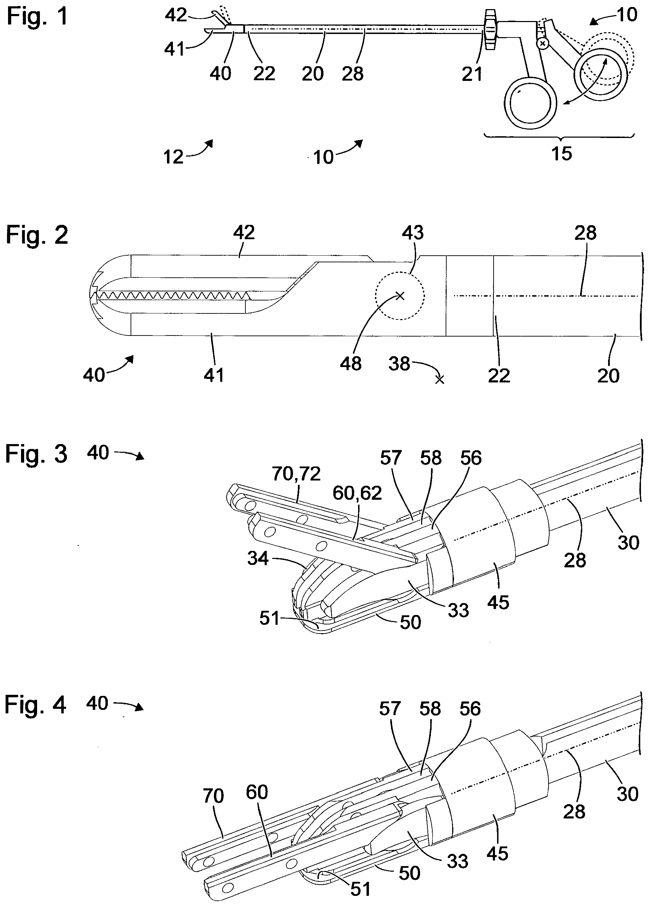

Figur 1 zeigt eine schematische Darstellung eines medizinischen Instruments 10, das insbesondere für mikroinvasive chirurgische Eingriffe ausgebildet ist. Das medizinische Instrument 10 umfasst an seinem proximalen Ende 11 eine Handhabungseinrichtung 15 und an seinem distalen Ende 12 ein Werkzeug 40. Ein gerader oder gekrümmter, starrer oder flexibler Schaft 20 verbindet die Handhabungseinrichtung 15 und das Werkzeug 40. Das proximale Ende 21 des Schafts 20 ist mit der Handhabungseinrichtung 15 verbunden, das distale Ende 22 des Schafts 20 ist mit dem Werkzeug 40 verbunden.

Zwei Teile der Handhabungseinrichtung 15 sind relativ zueinander bewegbar. Eines der beiden Teile ist über den Schaft 20 starr mit dem Werkzeug 40 verbunden, der andere Teil der Handhabungseinrichtung 15 ist mittels einer im Schaft 20 angeordneten Übertragungseinrichtung (insbesondere einer Übertragungsstange) mit einem schwenkbaren Maulteil 42 des Werkzeugs 40 gekoppelt. Eine manuell bewirkte Relativbewegung der beiden Teile der Handhabungseinrichtung 15 hat eine korrespondierende Relativbewegung des schwenkbaren Maulteils 42 relativ zu einem feststehenden Maulteil 41 des Werkzeugs 40 zur Folge.

Figur 2 zeigte eine schematische vergrößerte Darstellung eines Werkzeugs 40, das dem oben anhand der Figur 1 dargestellten Werkzeug ähnelt und an medizinischen Instrumenten verwendbar ist, die dem oben anhand der Figur 1 dargestellten ähneln.

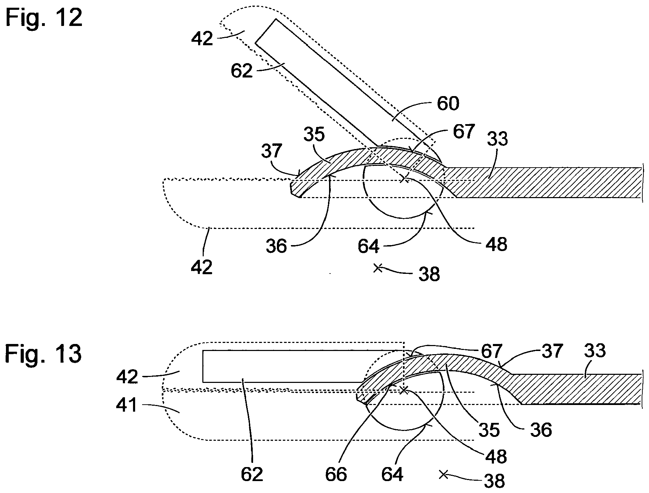

Das Werkzeug 40 ist mit dem distalen Ende 22 eines Schafts 20 lösbar mechanisch gekoppelt oder dauerhaft verbunden. Das Werkzeug 40 umfasst ein feststehendes Maulteil 41 und ein schwenkbares Maulteil 42. Das feststehende Maulteil 41 ist relativ zum Schaft 20 starr angeordnet. Das schwenkbare Maulteil 42 ist relativ zum feststehenden Maulteil 41 um eine Schwenkachse 48 senkrecht zur Zeichenebene der Figur 2 schwenkbar. Die Schwenkachse 48 ist durch ein Lager 43 definiert, das im Inneren des Werkzeugs 40 angeordnet und in Figur 2 durch in gestrichelter Linie angedeutet ist. Ein Krümmungsmittelpunkt 38 ist unten mit Bezug auf die Figuren 12 und 13 näher beschrieben.

Die Figuren 3 und 4 zeigen schematische axonometrische Darstellungen von Teilen des Werkzeugs 40 aus Figur 2. Das Werkzeug 40 umfasst einen Grundkörper 50 mit einer Grundplatte 51 mit einem stufenförmig profilierten Rand. Die Grundplatte 51 erstreckt sich im Wesentlichen parallel zur Längsachse eines mit dem Werkzeug 40 zu verbindenden Schafts und senkrecht zu den Zeichenebenen der Figuren 1 und 2. Senkrecht zur Grundplatte 51 sind ein erster Längssteg 56 und ein zweiter Längssteg 57 angeordnet. Die beiden Längsstege 56, 57 erstrecken sich parallel zur Längsachse eines mit dem Werkzeug 40 zu verbindenden Schafts und parallel zu den Zeichenebenen der Figuren 1 und 2. Zwischen den Längsstegen 56, 57 ist ein schmaler Arbeitskanal 58 vorgesehen, der sich wie die Längsstege 56, 57 parallel zur Längsachse 28 eines mit dem Werkzeug 40 zu verbindenden Schafts bis zum distalen Ende des Grundkörpers 50 erstreckt. Die beiden Längsstege 56, 57 sind nahe ihren von der Grundplatte 51 beabstandeten Rändern durch einen Verbindungsbolzen 59 verbunden. Der Verbindungsbolzen 59 erstreckt sich senkrecht zur Längsachse 28 eines mit dem Werkzeug 40 zu verbindenden Schafts und senkrecht zu den Zeichenebenen der Figuren 1 und 2.

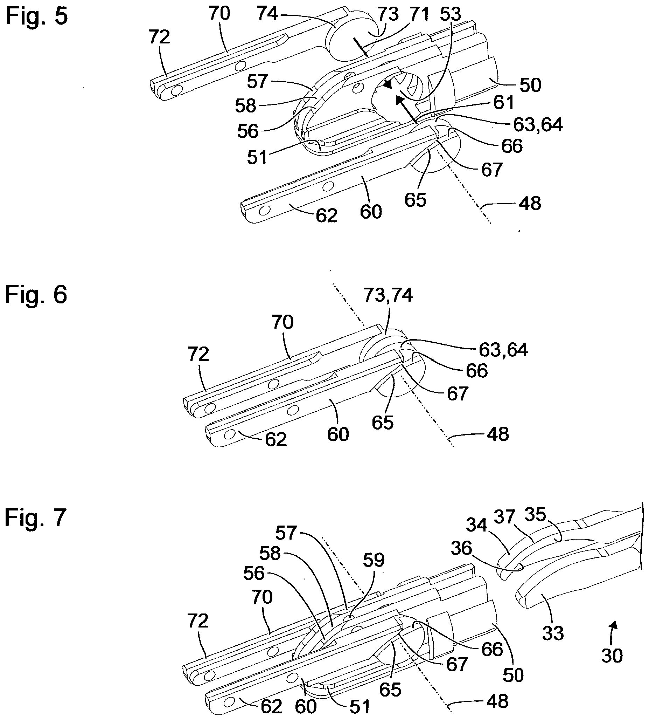

Ein erstes Achszapfenbauteil 60 und ein zweites Achszapfenbauteil 70 sind spiegelsymmetrisch zueinander ausgebildet und schwenkbar mit dem Grundkörper 50 verbunden. Merkmale der Lagerung der Achszapfenbauteile 60, 70 am Grundkörper 50 sind unten mit Bezug auf die Figuren 5 bis 7 näher beschrieben. Die Achszapfenbauteile 60, 70, insbesondere ein Holm 62 des ersten Achszapfenbauteils 60 und ein Holm 72 des zweiten Achszapfenbauteils 70, sind Bestandteile der in den Figuren 1 und 2 dargestellten schwenkbaren Maulteile 42.

Eine Übertragungseinrichtung 30 erstreckt sich im Schaft 20 (vgl. Figur 1) von der Handhabungseinrichtung 15 bis zum Werkzeug 40. Die Übertragungseinrichtung 30 umfasst am distalen Ende zwei parallele und spiegelsymmetrisch zueinander ausgebildete Holme 33, 34. In den Figuren 3 und 4 ist nur der erste Holm 33 deutlich erkennbar, der zweite Holm 34 ist weitgehend verdeckt. Die Holme 33, 34 am distalen Ende der Übertragungseinrichtung 30 sind beiderseits der Längsstege 56, 57 des Grundkörpers 50 angeordnet.

Am proximalen Ende des Grundkörpers 50 ist eine Hülse 45 angeordnet. Die Hülse 45 weist bei dem dargestellten Beispiel einen stufenförmig variierenden Querschnitt auf. Die Hülse 45 umgibt die Übertragungseinrichtung 30 bzw. dessen Holme 33, 34 ringförmig. Am proximalen Rand der Hülse 45 kann eine Kupplung zur lösbaren mechanischen Kopplung des Werkzeugs 40 mit einem distalen Ende 22 eines Schafts 20 (vgl. Figuren 1 und 2) angeordnet sein.

Jeder Holm 33, 34 weist einen gekrümmten Steg auf, der in eine gekrümmte Nut an einem der Achszapfenbauteile 60, 70 eingreift. Diese Stege an den Holmen 33, 34 und die Nuten an den Achszapfenbauteilen 60, 70 sind in den Figuren 3 und 4 nicht erkennbar, sondern werden unten mit Bezug auf die Figuren 5 bis 7, 12 und 13 beschrieben. Durch den Eingriff der Stege an den Holmen 33, 34 in die Nuten an den Achszapfenbauteilen 60, 70 sind eine Translationsbewegung der Übertragungseinrichtung 30 und eine Schwenkbewegung der Achszapfenbauteile 60, 70 miteinander gekoppelt.

In Figur 3 sind die Übertragungseinrichtung 30 in einer distalen Position und die Achszapfenbauteile 60, 70 bzw. deren Holme 62, 72 in zu der distalen Position der Übertragungseinrichtung 30 korrespondierenden offenen Positionen gezeigt. In Figur 4 sind die Übertragungseinrichtung 30 in einer proximalen Position und die Achszapfenbauteile 60, 70 bzw. deren Holme 62, 72 in zu der proximalen Position der Übertragungseinrichtung 30 korrespondierenden geschlossenen Positionen dargestellt.

Figur 5 zeigt eine schematische axonometrische Darstellung des Grundkörpers 50 und der Achszapfenbauteile 60, 70 vor dem Einsetzen der Achszapfenbauteil 60, 70 in den Grundkörper 50. Der Grundkörper 50 weist eine Ausnehmung 53 in Form einer Durchgangsbohrung senkrecht zur Längsachse 28 (vgl. Figuren 1 bis 4) und parallel zur Schwenkachse 48 des schwenkbaren Maulteils 42 (vgl. Figur 2) auf. Die Durchgangsbohrung 53 durchstößt beide Längsstege 56, 57 des Grundkörpers 50. Das erste Achszapfenbauteil 60 umfasst einen Achszapfen 63 mit einer schmalen ringförmigen Mantelfläche 64. Das zweite Achszapfenbauteil 70 umfasst einen Achszapfen 73 mit einer schmalen ringförmigen Mantelfläche 74.

Die Achszapfen 63, 73 können durch mittels Pfeilen angedeutete Einsetzbewegungen 61, 71 parallel zur Schwenkachse 48 in die Ausnehmung 53 am Grundkörper 50 eingesetzt werden. Die Ausnehmung 53 weist einen kreisförmigen Querschnitt auf, der so auf die Mantelflächen 64, 74 der Achszapfen 63, 73 abgestimmt ist, dass die Achszapfen 63, 73 nach dem Einsetzen 61, 71 in die Ausnehmung 53 spiel- und reibungsarm am Grundkörper geführt sind. Die Ausnehmung 53 im Grundkörper 50 und die in die Ausnehmung 53 eingreifenden Achszapfen 63, 73 bilden das bereits in Figur 2 angedeutete Gelenk bzw. Lager 43, das die Schwenkachse 48 des schwenkbaren Maulteils 42 definiert. Die Breiten der ringförmigen Mantelflächen 64, 74 der Achszapfen 63, 73 entsprechen im Wesentlichen den Wandstärken der Längsstege 56, 57 am Grundkörper 50.

Figur 6 zeigt eine weitere schematische axonometrische Darstellung der Achszapfenbauteile 60, 70 aus den Figuren 3 bis 5. Die Achszapfenbauteile 60, 70 sind in der relativen Position und dem Abstand dargestellt, die sie aufweisen, wenn die Achszapfen 63, 73 der Achszapfenbauteile 60, 70 vollständig in die Ausnehmung 53 am Grundkörper 50 (vgl. Figur 5) eingesetzt sind. Der Grundkörper 50 ist jedoch in Figur 6 nicht dargestellt. Die Achszapfen 63, 73 bzw. deren kreisförmige Stirnflächen weisen einen Abstand auf, der im Wesentlichen der Breite des Arbeitskanals 58 zwischen den Längsstegen 56, 57 am Grundkörper 50 (vgl. Figuren 3 bis 5) entspricht. Anders ausgedrückt greifen die Achszapfen 63, 73 nicht in den Arbeitskanal 58 ein bzw. schränken dessen Lumen bzw. Querschnitt nicht ein.

Figur 7 zeigt eine schematische axonometrische Darstellung des Grundkörpers 50 und der Achszapfenbauteile 60, 70. Die Achszapfen 63, 73 (vgl. Figuren 5 und 6) der Achszapfenbauteile 60, 70 sind in die Ausnehmung 53 (vgl. Figur 5) im Grundkörper 50 eingesetzt. Ferner ist das distale Ende der Übertragungseinrichtung 30 mit den Holmen 33, 34 dargestellt. Die Holme 33, 34 sind jedoch noch nicht in den Grundkörper 50 eingesetzt und mit den Achszapfenbauteilen 60, 70 gekoppelt.

In den Figuren 5 bis 7 ist eine gekrümmte Nut 65 im ersten Achszapfenbauteil 60 erkennbar. Die gekrümmte Nut 65 weist zwei im Wesentlichen parallele gekrümmte Gleitflächen 66, 67 auf, die jedoch unterschiedlich breit sind. Die Nut 65 erstreckt sich im Wesentlichen vollständig innerhalb des Bereichs des Achszapfens 63, genauer gesagt innerhalb eines kreiszylindrischen Raumbereichs, dessen Mantelfläche abschnittsweise den Mantelflächen 64, 74 der Achszapfen 63, 73 der Achszapfenbauteile 60, 70 (vgl. Figuren 5 und 6) entspricht. Das zweite Achszapfenbauteil 70 ist spiegelsymmetrisch zum ersten Achszapfenbauteil 60 ausgebildet und weist insbesondere eine der Nut 65 am ersten Achszapfenbauteil 60 spiegelsymmetrisch entsprechende gekrümmte Nut auf, die in den Figuren 5 bis 7 nicht erkennbar ist.

Am zweiten Holm 34 am distalen Ende der Übertragungseinrichtung 30 ist ein gekrümmter Steg 35 mit einer ersten Gleitfläche 36 und einer zweiten Gleitfläche 37 erkennbar. Der erste Holm 33 weist einen zum gekrümmten Steg 35 am zweiten Holm 34 spiegelsymmetrischen gekrümmten Steg auf. Die Breiten und die Krümmungen der gekrümmten Nuten 65 an den Achszapfenbauteilen 60, 70 und die Breiten und Krümmungen der Stege 35 an den Holmen 33, 34 der Übertragungseinrichtung 30 entsprechen einander so, dass die Stege 35 an den Holmen 33, 34 spiel- und reibungsarm in den Nuten 65 an den Achszapfenbauteilen 60, 70 geführt sind, wenn das distale Ende der Übertragungseinrichtung 30 in den Grundkörper 50 und die Achszapfenbauteile 60, 70 eingesetzt ist.

Nach dem Einsetzen der Achszapfen 63, 73 in die Ausnehmung 53 im Grundkörper 50 und dem Koppeln der Holme 33, 34 mit den Achszapfenbauteilen 60, 70 durch Einführen der gekrümmten Stege 35 in die gekrümmte Nut 65 werden die Holme 62, 72 der Achszapfenbauteile 60, 70 durch einen in den Figuren 3 bis 7 nicht dargestellten Grundkörper des schwenkbaren Maulteils 42 (vgl. Figuren 1 und 2) miteinander verbunden. Der Grundkörper des schwenkbaren Maulteils 42 weist insbesondere Bohrungen oder Nuten auf, in die die Holme 62, 72 der Achszapfenbauteile 60, 70 eingeführt werden. Durch den erwähnten Grundkörper des schwenkbaren Maulteils 42 werden die Achszapfenbauteile 60, 70 in ihrer in den Figuren 3, 4, 6 und 7 dargestellten relativen Position und damit die Achszapfen 63, 73 in der Ausnehmung 53 im Grundkörper 50 gehalten.

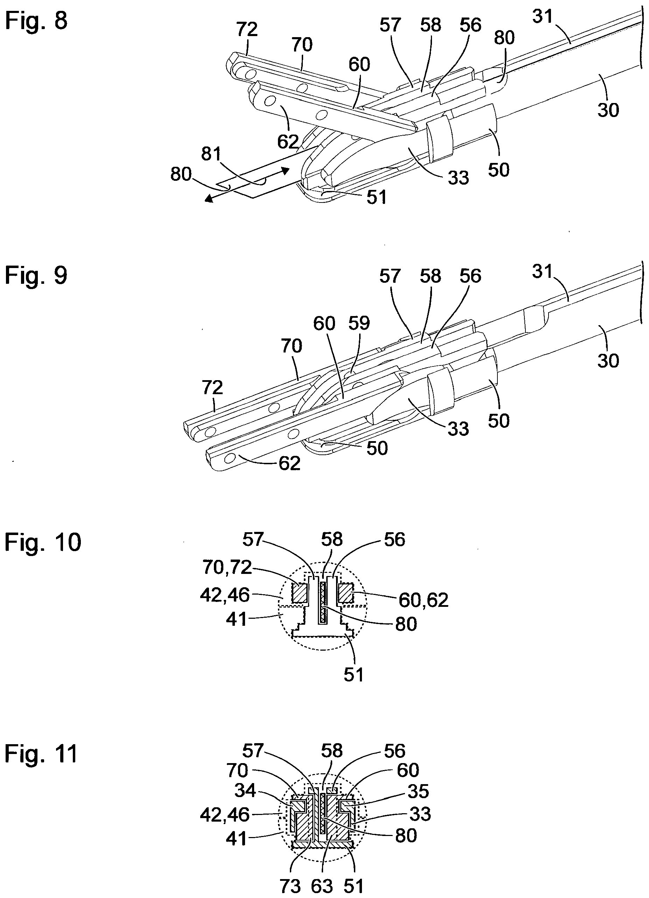

Die Figuren 8 und 9 zeigen schematische axonometrische Darstellungen des Grundkörpers 50, der Achszapfenbauteile 60, 70 und der Übertragungseinrichtung 30. Die Darstellungen in den Figuren 8 und 9 entsprechen auch hinsichtlich der Positionen der Übertragungseinrichtung 30 und der Achszapfenbauteile 60, 70 den Darstellungen in den Figuren 3 bzw. 4. Im Unterschied zu den Darstellungen in den Figuren 3 und 4 ist die Hülse 45 am proximalen Ende des Grundkörpers 50 in den Figuren 8 und 9 nicht dargestellt. Dadurch ist der erste Holm 33 in größerem Umfang sichtbar. In den Figuren 5 bis 9 ist erkennbar, dass der Grundkörper 50 parallel zum Arbeitskanal 58 und außen an die beiden Längsstege 56, 57 angrenzend zwei Nuten aufweist, in denen die Holme 33, 34 (vgl. Figur 7) geführt sind.

In Figur 8 ist ferner ein Skalpell 80 dargestellt, das im Arbeitskanal 58 des Grundkörpers 50 in Richtung 81 parallel zur Längsachse 28 (vgl. Figuren 1 bis 4) verschiebbar angeordnet ist. Proximal des Grundkörpers 50 liegt das Skalpell 80 in einer Nut 31 in der Übertragungseinrichtung 30, die den Arbeitskanal 58 nach proximal fortsetzt. Insbesondere erstreckt sich das Skalpell 80 bis zur Handhabungseinrichtung 15 (vgl. Figur 1), um eine Bewegung des Skalpells 80 von der Handhabungseinrichtung 15 aus zu ermöglichen. Entsprechende Einrichtungen an der Handhabungseinrichtung 15 sind in Figur 1 nicht dargestellt.

Die Figuren 10 und 11 zeigen schematische Darstellungen von Querschnitten durch den Grundkörper 50, die Achszapfenbauteile 60, 70 und das Skalpell 80. In Figur 11 sind ferner Querschnitte der Holme 33, 34 dargestellt. Die Konturen der Maulteile 41, 42 sind in gestrichelten Linien angedeutet. In beiden Figuren 10 und 11 ist das schwenkbare Maulteil 42 mit den Achszapfenbauteilen 60, 70 in der auch in den Figuren 2, 4, 7 und 9 gezeigten geschlossenen Position dargestellt.

Die Schnittebenen der Figuren 10 und 11 sind senkrecht zur Längsachse 28 (vgl. Figuren 1 bis 4) und zu den Zeichenebenen der Figuren 1 und 2. Die Schnittebene der Figur 10 liegt distal des Verbindungsbolzens 59 zwischen den Längsstegen 56, 57 des Grundkörpers 50 (vgl. Figuren 4 und 9). Die Schnittebenen der Figur 11 enthält die Schwenkachse 48 des schwenkbaren Maulteils 42 (vgl. Figuren 2 und 5 bis 7).

In den Figuren 10 und 11 ist jeweils der Aufbau des Grundkörpers 50 mit der in den Figuren 10 und 11 horizontal angeordneten Grundplatte 51 und den in den Figuren 10 und 11 vertikal angeordneten Längsstegen 56, 57 erkennbar. Ferner sind der Arbeitskanal 58 zwischen den Längsstegen 56, 57 und das Skalpell 80 im Arbeitskanal 58 erkennbar. Die Schnittebene der Figur 10 schneidet die beiden Holme 62, 72 der Achszapfenbauteile 60, 70. Die Schnittebene der Figur 11 schneidet die Achszapfen 63, 73 der Achszapfenbauteile 60, 70 und die Holme 33, 34 der Übertragungseinrichtung 30 (vgl. Figuren 3, 4, 8 und 9). In dem in Figur 11 dargestellten Querschnitt ist erkennbar, wie die Stege 35 an den Holmen 33, 34 in die korrespondierenden Nuten an den Achszapfenbauteilen 60, 70 eingreifen.

Ferner ist erkennbar, wie die Achszapfen 63, 73 in die Längsstege 56, 57 des Grundkörpers 50 eingreifen. Dabei sind in Figur 11 an den Achszapfen 63, 73 zwei unterschiedliche Varianten dargestellt. Der Achszapfen 63 des ersten Achszapfenbauteils 60 greift in eine als Durchgangsbohrung ausgebildete korrespondierende Ausnehmung im ersten Längssteg 56 ein und reicht bis zum Arbeitskanal 58. Der Achszapfen 73 am zweiten Achszapfenbauteil 70 greift in eine als Sackbohrung ausgebildete Ausnehmung am zweiten Längssteg 57 des Grundkörpers 50 ein. Der Achszapfen 73 des zweiten Achszapfenbauteils 70 ist deshalb durch einen dünnen Bereich des zweiten Längsstegs 57 vom Arbeitskanal 58 getrennt. Abweichend von der Darstellung in Figur 11 weist ein Werkzeug insbesondere zwei spiegelsymmetrische Achszapfenbauteile 60, 70 und zwei spiegelsymmetrische Längsstege 56, 57 auf. Der Eingriff beider Achszapfen in die Längsstege 56, 57 entspricht damit insbesondere entweder dem in Figur 11 dargestellten ersten Achszapfen 63 in den ersten Längssteg 56 oder dem in Figur 11 dargestellten zweiten Achszapfen 73 in den zweiten Längssteg 57.

In den Figuren 10 und 11 ist ferner der bereits in der Beschreibung der Figuren 5 bis 7 erwähnte Grundkörper 46 erkennbar, der die Achszapfenbauteile 60, 70 in ihren in den Figuren 3, 4, 6 und 7 dargestellten relativen Position und damit die Achszapfen 63, 73 in der Ausnehmung 53 im Grundkörper 50 hält. Dazu weist der Grundkörper 46 insbesondere in Figur 10 erkennbare Nuten auf, in denen die Holme 62, 72 der Achszapfenbauteile 60, 70 formschlüssig gehalten sind.

Die Figuren 12 und 13 zeigen schematische Darstellungen eines Schnitts durch den ersten Holm 33 und das erste Achszapfenbauteil 60 (vgl. Figuren 3 bis 11). Die Schnittebenen der Figuren 12 und 13 sind parallel zur Längsachse 28 (vgl. Figuren 1 bis 4), zu den Zeichenebenen der Figuren 1 und 2 und senkrecht zur Schwenkachse 48 des schwenkbaren Maulteils 42 (vgl. Figuren 2 und 5 bis 7) sowie zu den Schnittebenen der Figuren 10 und 11. Die Schnittebenen der Figuren 12 und 13 sind so angeordnet, dass sie die Nut 65 am ersten Achszapfenbauteil 60 und den Steg 35 am ersten Holm 33 schneiden. In gestrichelten Linien sind die Konturen des feststehenden Maulteils 41 und des schwenkbaren Maulteils 42 angedeutet.

In Figur 12 sind der Holm 33 in der distalen Position und das schwenkbare Maulteil 42 mit dem Achszapfenbauteil 60 in der offenen Position, die auch in den Figuren 3 und 8 gezeigt sind, dargestellt. In Figur 13 sind der Holm 33 in der proximalen Position und das schwenkbare Maulteil 42 mit dem Achszapfenbauteil 60 in der geschlossenen Position, die auch in den Figuren 4 bis 7 und 9 bis 11 gezeigt sind, dargestellt. Ferner ist in den Figuren 12 und 13 der Krümmungsmittelpunkt 38 des gekrümmten Stegs 35 am Holm 33 (und damit der Gleitflächen 36, 37 am gekrümmten Steg 35) und der gekrümmten Nut 65 (vgl. Figuren 5 bis 7) am Achszapfenbauteil 60 (und damit der Gleitflächen 66, 67 der gekrümmten Nut) dargestellt. Der Krümmungsmittelpunkt 38 liegt außerhalb des Werkzeugs 40 bzw. seiner in den Figuren 12 und 13 durch gestrichelte Linien angedeuteten Konturen.

### Bezugszeichen

- 10: medizinisches Instrument
- 11: proximales Ende des medizinischen Instruments 10
- 12: distales Ende des medizinischen Instruments 10
- 15: Handhabungseinrichtung
- 20: Schaft des medizinischen Instruments 10
- 21: proximales Ende des Schafts 20
- 22: distales Ende des Schafts 20
- 28: Längsachse des Schafts 20
- 30: Übertragungseinrichtung
- 31: Längsnut in der Übertragungseinrichtung 30
- 32: distales Ende der Übertragungseinrichtung 30
- 33: erster Holm am distalen Ende der Übertragungseinrichtung 30
- 34: zweiter Holm am distalen Ende der Übertragungseinrichtung 30
- 35: gekrümmter Steg an einem Holm 33, 34
- 36: erste Gleitfläche am Steg 35
- 37: zweite Gleitfläche am Steg 35
- 38: Krümmungsmittelpunkt des Stegs 35
- 40: Werkzeug
- 41: feststehendes Maulteil des Werkzeugs 40
- 42: schwenkbares Maulteil des Werkzeugs 40
- 43: Lager
- 45: Hülse
- 46: Grundkörper des schwenkbaren Maulteils 42
- 48: Schwenkachse des schwenkbar gelagerten Maulteils 40
- 50: Grundkörper des Werkzeugs 40
- 51: Grundplatte des Grundkörpers 50
- 53: Ausnehmung am Grundkörper 50
- 56: erster Längssteg am Grundkörper 50
- 57: zweiter Längssteg am Grundkörper 50
- 58: Arbeitskanal zwischen den Längsstegen 56, 57 des Grundkörpers 50
- 59: Verbindungsbolzen
- 60: erstes Achszapfenbauteil des schwenkbar gelagerten Maulteils 42
- 61: Einsetzbewegung
- 62: Holm des ersten Achszapfenbauteils 60
- 63: Achszapfen am ersten Achszapfenbauteil 60
- 64: Mantelfläche des Achszapfens 63
- 65: Nut am ersten Achszapfenbauteil 60
- 66: erste Gleitfläche an der Nut 65
- 67: zweite Gleitfläche an der Nut 65
- 70: zweites Achszapfenbauteil des schwenkbar gelagerten Maulteils 42
- 71: Einsetzbewegung
- 72: Holm des zweiten Achszapfenbauteils 70
- 73: Achszapfen am zweiten Achszapfenbauteil 70
- 74: Mantelfläche des Achszapfens 73
- 80: Skalpell
- 81: Bewegungsrichtung des Skalpells 80

## Patentansprüche

1. **Werkzeug** (40) für ein distales Ende (22) oder an einem distalen Ende (22) eines Schafts (20) eines medizinischen Instruments (10), mit:
einem **Grundkörper** (50);
einem **Maulteil** (42), das relativ zum Grundkörper (50) um eine Schwenkachse (48) schwenkbar ist;
einem **Lager** (43), das die Schwenkachse (48) definiert,
wobei das Lager (43) zwei voneinander beabstandete Achszapfen (63, 73) mit je einer zur Schwenkachse (48) rotationssymmetrischen Mantelfläche (64, 74) umfasst,
**dadurch gekennzeichnet, dass** das Maulteil (42) zwei **Achszapfenbauteile** (60, 70) mit je einem der Achszapfen (63, 73) umfasst.

2. Werkzeug (40) nach dem vorangehenden Anspruch, ferner mit:
einer **Übertragungseinrichtung** (30) zum Übertragen einer Kraft zum Werkzeug (40);
wobei die Übertragungseinrichtung (30) und das schwenkbare Maulteil (42) aneinander anliegende **gekrümmte Gleitflächen** (36, 37, 66, 67) aufweisen, die dazu ausgebildet sind, eine Translationsbewegung der Übertragungseinrichtung (30) mit einer Schwenkbewegung des schwenkbaren Maulteils (42) zu koppeln,
wobei ein **Krümmungsmittelpunkt** (38) der gekrümmten Gleitflächen (36, 37, 66, 67) **außerhalb** des Werkzeugs (40) liegt.

3. Werkzeug (40) nach einem der vorangehenden Ansprüche, bei dem an zumindest einem der zwei Achszapfen (63, 73) eine Nut (65) innerhalb eines kreiszylindrischen Raumbereichs vorgesehen ist, wobei die Mantelflächen (64, 74) der Achszapfen (63, 73) auf einer Mantelfläche des kreiszylindrischen Raumbereichs liegen.

4. Werkzeug (40) nach einem der vorangehenden Ansprüche, bei dem die Achszapfenbauteile (60, 70) **nach** einem **Einsetzen** der Achszapfen (63, 73) in korrespondierende Ausnehmungen (53) miteinander **starr verbunden** wurden.

5. Werkzeug (40) nach dem vorangehenden Anspruch, bei dem die Achszapfenbauteile (60, 70) **mittels eines Grundkörpers** (46) des schwenkbaren Maulteils (42) miteinander starr verbunden sind.

6. Werkzeug (40) nach einem der vorangehenden Ansprüche, ferner mit:
einem **Arbeitskanal** (58), in dem ein Skalpell (80) oder ein anderes Wirkelement zwischen einer proximalen und einer distalen Position verschiebbar ist,
wobei der Arbeitskanal (58) **zwischen den Achszapfen** (63, 73) angeordnet ist.

7. Werkzeug (40) nach dem vorangehenden Anspruch,
wobei der Grundkörper (50) des Werkzeugs (40) zwei parallele **Wände** (56, 57) umfasst, zwischen denen ein Abschnitt des Arbeitskanals (58) angeordnet ist, wobei jede der zwei parallelen Wände (56, 57) eine Ausnehmung (53) aufweist, in die einer der zwei Achszapfen (63, 73) eingreift.

8. Werkzeug (40) nach dem vorangehenden Anspruch, bei dem die zwei parallelen Wände (56, 57) als **Stege an einer Grundplatte** (51) ausgebildet sind.

9. Werkzeug (40) nach einem der vorangehenden Ansprüche, bei dem
das Lager (43) ferner zu den Achszapfen (62, 72) **korrespondierende Ausnehmungen** (53) umfasst,
die **Achszapfen** (63, 73) **von außen** in die Ausnehmungen (53) eingreifen.

10. **Medizinisches Instrument** (10) mit einem Werkzeug (40) nach einem der vorangehenden Ansprüche.

## Claims

1. Tool (40) for a distal end (22) or on a distal end (22) of a shank (20) of a medical instrument (10), said tool (40) having:
a main body (50);
a jaw part (42), which is pivotable relative to the main body (50) about a pivot axis (48);
a bearing (43), which defines the pivot axis (48), wherein the bearing (43) comprises two journals (63, 73), which are spaced apart from each other and each have a jacket surface (64, 74) that is rotationally symmetrical with respect to the pivot axis (48),
**characterized in that** the jaw part (42) comprises two journal components (60, 70), each of them with one of the journals (63, 73).

2. Tool (40) according to the preceding claim, also having:
a transmission mechanism (30) for transmitting a force to the tool (40);
wherein the transmission mechanism (30) and the pivotable jaw part (42) have curved sliding faces (36, 37, 66, 67), which bear on each other and are designed to couple a translation movement of the transmission mechanism (30) to a pivoting movement of the pivotable jaw part (42),
wherein a centre of curvature (38) of the curved sliding faces (36, 37, 66, 67) lies outside the tool (40).

3. Tool (40) according to one of the preceding claims, in which a groove (65) is provided within a circular cylindrical spatial area on at least one of the two journals (63, 73), wherein the jacket surfaces (64, 74) of the journals (63, 73) lie on a jacket surface of the circular cylindrical spatial area.

4. Tool (40) according to one of the preceding claims, in which the journal components (60, 70) were rigidly connected to each other after insertion of the journals (63, 73) into corresponding recesses (53).

5. Tool (40) according to the preceding claim, in which the journal components (60, 70) are rigidly connected to each other by means of a main body (46) of the pivotable jaw part (42).

6. Tool (40) according to one of the preceding claims, also having:
a work channel (58), in which a scalpel (80) or another effecting element is slidable between a proximal position and a distal position,
wherein the work channel (58) is arranged between the journals (63, 73).

7. Tool (40) according to the preceding claim,
wherein the main body (50) of the tool (40) comprises two parallel walls (56, 57), between which a portion of the work channel (58) is arranged,
wherein each of the two parallel walls (56, 57) has a recess (53), in which one of the two journals (63, 73) engages.

8. Tool (40) according to the preceding claim, in which the two parallel walls (56, 57) are designed as webs on a base plate (51).

9. Tool (40) according to one of the preceding claims, in which the bearing (43) moreover comprises recesses (53) corresponding to the journals (62, 72), and the journals (63, 73) engage in the recesses (53) from the outside.

10. Medical instrument (10) having a tool (40) according to one of the preceding claims.

## Revendications

1. Outil (40) pour une extrémité distale (22) ou au niveau d'une extrémité distale (22) d'une tige (20) d'un instrument médical (10), comprenant :
un corps de base (50) ;
une partie de mâchoire (42) qui peut pivoter par rapport au corps de base (50) autour d'un axe de pivotement (48) ;
un palier (43) qui définit l'axe de pivotement (48).
le palier (43) comprenant deux tourillons d'axe (63, 73) espacés l'un de l'autre ayant chacun une surface d'enveloppe à symétrie de révolution (64, 74) par rapport à l'axe de pivotement (48), **caractérisé en ce que** la partie de mâchoire (42) comprend deux composants de tourillon d'axe (60, 70) ayant chacun l'un des tourillons d'axe (63, 73).

2. Outil (40) selon la revendication précédente, comprenant en outre :
le dispositif de transfert (30) pour transférer une force à l'outil (40) ;
le dispositif de transfert (30) et la partie de mâchoire pivotante (42) présentant des surfaces de glissement courbes (36, 37, 66, 67) s'appliquant l'une contre l'autre, qui sont réalisées de manière à accoupler un mouvement de translation du dispositif de transfert (30) à un mouvement de pivotement de la partie de mâchoire pivotante (42),
un centre de courbure (38) des surfaces de glissement courbes (36, 37, 66, 67) étant situé à l'extérieur de l'outil (40).

3. Outil (40) selon l'une quelconque des revendications précédentes, dans lequel une rainure (65) est prévue au niveau d'au moins l'un des deux tourillons d'axe (63, 73) à l'intérieur d'une région spatiale cylindrique circulaire, les surfaces d'enveloppe (64, 74) des tourillons d'axe (63, 73) étant situées sur une surface d'enveloppe de la région spatiale cylindrique circulaire.

4. Outil (40) selon l'une quelconque des revendications précédentes, dans lequel les composants des tourillons d'axe (60, 70) étaient connectés rigidement l'un à l'autre après l'insertion des tourillons d'axe (63, 73) dans des évidements correspondants (53).

5. Outil (40) selon la revendication précédente, dans lequel les composants des tourillons d'axe (60, 70) sont connectés rigidement l'un à l'autre au moyen d'un corps de base (46) de la partie de mâchoire pivotante (42).

6. Outil (40) selon l'une quelconque des revendications précédentes, comprenant en outre :
un canal de travail (58), dans lequel un scalpel (80) ou un autre élément fonctionnel peut être déplacé entre une position proximale et une position distale,
le canal de travail (58) étant disposé entre les tourillons d'axe (63, 73).

7. Outil (40) selon la revendication précédente,
dans lequel le corps de base (50) de l'outil (40) comprend deux parois parallèles (56, 57) entre lesquelles est disposée une portion du canal de travail (58),
chacune des deux parois parallèles (56, 57) présentant un évidement (53) dans lequel s'engage l'un des deux tourillons d'axe (63, 73).

8. Outil (40) selon la revendication précédente, dans lequel les deux parois parallèles (56, 57) sont réalisées sous forme de nervures au niveau d'une plaque de base (51).

9. Outil (40) selon l'une quelconque des revendications précédentes, dans lequel le palier (43) comprend en outre des évidements (53) correspondant aux tourillons d'axe (62, 72), les tourillons d'axe (63, 73) viennent en prise depuis l'extérieur dans les évidements (53).

10. Instrument médical (10) comprenant un outil (40) selon l'une quelconque des revendications précédentes.
